# EUROPEAN PATENT SPECIFICATION

(11) **EP 2 941 551 B1**
(45) Date of publication and mention of the grant of the patent: **25.04.2018**
(21) Application number: 13814156.9
(22) Date of filing: 20.12.2013
(51) Int. Cl.: F01N 3/20

(54) **METHOD AND SYSTEM FOR GENERATING POWER ON BOARD A VEHICLE**
VERFAHREN ZUR ENERGIEERZEUGUNG AN BORD EINES FAHRZEUGS
PROCÉDÉ DE GÉNÉRATION D'ÉNERGIE À BORD D'UN VÉHICULE

(30) Priority: 21.12.2012 EP 12199278
(43) Date of publication of application: 11.11.2015
(73) Proprietor: Plastic Omnium Advanced Innovation and Research, 1120 Bruxelles (BE)
(72) Inventor: DOUGNIER, Francois, B-3190 Boortmeerbeek (BE); VAN SCHAFTINGEN, Jules-Joseph, B-1300 Wavre (BE); MADOUX, Dominique, B-7611 Rumes (BE); WOUTERS, Paul, B-1800 Vilvoorde (BE)
(74) Representative: de la Bigne, Guillaume Michel Marie
(86) International application number: PCT/EP2013/077851
(87) International publication number: WO 2014/096426

(56) References cited:
- EP-A1- 1 977 817
- DE-A1-102008 042 735
- US-A1- 2003 219 371
- US-A1- 2004 115 110
- US-A1- 2011 302 909

## Description

The present invention relates to the generation of power on board a vehicle. For example, the present invention can apply in vehicles powered by fuel cells or internal combustion engines or in vehicles equipped with an hybrid power train such as Plug-in vehicles using a fuel cell to recharge the electrical batteries.

Legislation on vehicle and truck emissions imposes steadily most stringent emissions; in particular, targets for CO₂ emissions have been drastically reduced in recent years and will continue to do so. At the same time, the authorities in charge of some critically polluted cities are imposing restrictions of the traffic or are considering doing so, in order to limit the generation of CO₂ emissions within specific environments. Known techniques to do so include the use of electrical vehicles or hybrid vehicles or plug-in hybrid vehicles. However the driving range of these vehicles is typically quite limited. Recharge of batteries is time consuming and a lot longer than the refuelling operation typically necessary for vehicles using hydrocarbon fuels such as gasoline, diesel or natural gas. Activation of an internal combustion engine based on hydrocarbon to recharge the batteries and to extend the driving range of a vehicle is also generating CO₂ emissions. Document US 2003/0219371 discloses a method of generating energy from a urea based composition.

In view of the above-mentioned disadvantage, there exists a need for an improved method for generating power to recharge batteries (i.e. energy accumulator) of a vehicle.

An object of the present invention is to solve this above-mentioned problem by proposing a method for generating power on board a vehicle. According to one aspect of the present invention, the method comprises:
- generating a gas by chemically decomposing an ammonia precursor in a biochemical decomposition unit mounted on board the vehicle and storing at least one protein component adapted to decompose said ammonia precursor;
- converting said gas into power in at least one power generator mounted on board the vehicle.

Thus, it is proposed an in situ power generation mechanism. In other words, the generation of power takes place on board the vehicle. More precisely, the power generation mechanism according to the invention is based on the decomposition of an ammonia precursor. Such decomposition is obtained by using a biochemical decomposition unit mounted on board the vehicle. The biochemical decomposition unit according to the invention stores one or several protein component(s) that catalyze a chemical reaction. More precisely, the protein component(s) is(are) adapted to catalyze the hydrolysis (i.e. decomposition) of the ammonia precursor. This decomposition results in the generation of a gas.

In a particular embodiment, the protein component(s) is(are) adapted to decompose the ammonia precursor into ammonia gas.

In another particular embodiment, the protein component(s) is(are) adapted to decompose the ammonia precursor into hydrogen gas.

In a particular embodiment, the hydrogen gas is generated as follows:
- first, the ammonia precursor is decomposed with a first protein component for generating an ammonia gas;
- then, the generated ammonia gas is decomposed with a second protein component for generating the hydrogen gas.

Once generated, the gas can be stored or can be directly converted into power in a power generator (or a plurality of power generators) mounted on board the vehicle.

In the present document, the term "power generator" is understood to mean any component, device or system adapted to produce power from a gas.

In a particular embodiment, the power generator is a fuel cell. For example, the fuel cell can be fed with the gas (ammonia gas, hydrogen gas,...) generated out of the biochemical decomposition unit. The fuel cell can be used to produce electrical power. In a particular embodiment, the electrical power produced by the fuel cell can fed an electrical motor for generating mechanical power.

In another particular embodiment, the power generator is an internal combustion engine. In this particular case, the generated gas is used as a fuel in the combustion engine.

Advantageously, the power generated at the output of the power generator (for example, an electrical power) is used to recharge, for example, batteries mounted on board the vehicle.

In a particular embodiment, the gas generated by the biochemical decomposition unit is directed (i.e. transmitted) to at least one solid absorbing matrix where it is stored thereon by sorption. Advantageously, the solid absorbing matrix is stored in a container (or tank) (called hereafter matrix storage container) mounted on the vehicle. According to the present invention, if it is desired to transmit gas (ammonia gas, hydrogen gas,...) to the power generator, then the solid absorbing matrix is heated so as to release the gas stored thereon. Thus, the container storing the solid absorbing matrix acts as a gas buffer so as to supply the power generator with gas. Thus, the supply of gas to the power generator according to the invention is simple, faster and safer.

In a preferred embodiment, a predetermined amount of ammonia precursor is stored on board the vehicle. For example, during vehicle operation, it is calculated a desired amount of ammonia precursor to be injected into the biochemical decomposition unit. Such calculation can be made as a function of information relative to the amount of ammonia gas (or hydrogen gas, or any suitable type of gas) that has been consumed (i.e. amount of gas transmitted to the power generator). In a particular embodiment, such information may derive from data provided by a temperature sensor, a pressure sensor or a flow meter, or any combination of these sensors. In another particular embodiment, such information may derive from data provided by a device configured to measure the concentration of ammonia stored in the solid absorbing matrix. In another particular embodiment, this information may be derived from an estimation of the consumption of ammonia.

Advantageously, the protein component (stored in the biochemical decomposition unit) comprises at least one enzyme. In particular, thermophile-type enzymes are well suited. In a preferred embodiment, the biochemical decomposition unit can store urease. Urease can be stored in any suitable manner. For example, in a first embodiment urease can be immobilized in different layers of resin. In a second embodiment urease can be fixed on membranes.

In a particular embodiment, the ammonia precursor is an aqueous urea solution.

The terms "urea solution" are understood to mean any, generally aqueous, solution containing urea. The invention gives good results with eutectic water/urea solutions for which there is a quality standard: for example, according to the standard ISO 22241, in the case of the AdBlue® solution (commercial solution of urea), the urea content is between 31.8 % and 33.2 % (by weight) (i.e. 32.5 +/- 0.7 wt%) hence an available amount of ammonia between 18.0 % and 18.8 %. The invention may also be applied to aqueous urea solutions which are richer in urea than the eutectic solution: for instance, so called AdBlue® 40 containing about 40% in weight of urea can be used, providing a larger driving range than the eutectic solution. The invention may also be applied to the urea/ammonium formate mixtures, also in aqueous solution, sold under the trade name Denoxium™ and of which one of the compositions (Denoxium-30) contains an equivalent amount of ammonia to that of the AdBlue® solution. The latter have the advantage of only freezing from -30°C onwards (as opposed to - 11°C), but have the disadvantages of corrosion problems linked to the possible release of formic acid. The invention can also apply to guanidinium formate. The present invention is particularly advantageous in the context of eutectic water/urea solutions, which are widely available in gas stations.

In a particular embodiment, the urea solution can be partially stored in a chamber located within the biochemical decomposition unit, before it is chemically decomposed.

In another particular embodiment, the solid absorbing matrix is stored in a first tank and the ammonia precursor is stored in a second tank. Advantageously, the second tank (storing the ammonia precursor) is connected in a communicating manner to said biochemical decomposition unit, and said biochemical decomposition unit is connected in a communicating manner to the first tank (storing the solid absorbing matrix). In a first embodiment, the first tank and the second tank can be separate storage tanks. In a second embodiment, the first tank and the second tank can be two separate chambers of a same container.

It should be noted that it exists well known refilling standards and systems for ammonia precursor, in particular for the AdBlue® solution (commercial solution of urea). The refilling of the storage tank of the ammonia precursor is trivial. For example, this can be achieved by using available standard-designed nozzle and/or bottles with dedicated interfaces.

In a particular embodiment, the biochemical decomposition unit can be located below the storage tank of the ammonia precursor. In this particular embodiment, a stream (i.e. part) of the ammonia precursor can be transported towards the biochemical decomposition unit by gravity.

According to the invention, if the ammonia precursor generates water by thermal decomposition, this water is separated from the ammonia, collected and preferably prevented from being stored on the solid absorbing matrix. Separation of the water from the ammonia, and generally from the eventual other thermal decomposition products (generally gases like CO₂), can be made using a liquid-vapour separator unit. For example, the liquid-vapour separator unit can comprise (or be) a condenser or one or several membranes like disclosed in US patent 4758250 for instance, which is a polymeric membrane. The condenser may be a specific one comprising a specific shaped tube having different parts at different temperatures (as described in example 2 below); a phase change material, or any other means for cooling and condensing the gases. Alternatively, the condenser may be part of a device already onboard the vehicle, for instance: part of the vehicle air conditioning system. This also applies to the case of hydrogen.

In a particular embodiment, the water collected may be vaporised by using the heat rejected by the power generator and/or at least part of it can be stored for instance to be available for dissolving excess ammonia that would pressurize unduly the storage tank of the solid absorbing matrix (see embodiment with pressure relief valve described below).

The ammonia or hydrogen is transferred (to the power generator) using a gas line which may comprise a non return valve close to the metering point.

The method according to the invention uses two separate storage tanks: one for the ammonia precursor and one for the solid absorbing matrix which stores ammonia by sorption. As described in patent application WO 2006/012903, metal ammine salts (preferably alkaline earth metal chlorides) can be used as solid storage media for ammonia.

Advantageously, the biochemical decomposition unit is equipped with a heater. Such heater can provide the optimum temperature for the desired activity of the enzyme or protein. For example, the heater can be configured to maintain within the biochemical decomposition unit a temperature range between 30°C and 60°C.

More generally, the heater is a chamber whose temperature is controlled within predetermined ranges; in case the predetermined range falls below the temperature of the environment, cooling means will also be made available within the heater. In other words, the heater can either be controlled so as to rise up the temperature within the chamber or controlled so as to cool down the temperature within the chamber.

In a particular embodiment, the heater is configured to work within at least one predetermined temperature range corresponding to the activation of the protein component when conversion is needed, and within at least another predetermined temperature range corresponding to the preservation of the protein component, so as to extend its lifetime.

In a particular embodiment of the invention, the heater can comprise resistive heating elements. These resistive heating elements may be metallic heating filaments (wires), flexible heaters, (that is to say heaters comprising one or more resistive track(s) affixed to a film or placed between two films (that is to say two substantially flat supports, the material and thickness of which are such that they are flexible)) or any other type of resistive elements that have a shape, size and flexibility suitable for being inserted into and/or wound around the components of the power generation system. PTC (Positive Temperature Coefficient) elements are more particularly suitable for heating.

In another particular embodiment of the invention, the heater uses the dissipated heat of the power generator (for instance, a flow of the cooling system from fuel cell or internal combustion engine) and/or exhaust line (gases) for heating the biochemical decomposition unit.

According to the invention, after generating the ammonia gas (or hydrogen gas), it can be compressed by means of a gas pressurisation unit. The function of this gas pressurisation unit is to compress the ammonia gas (or hydrogen gas) to a suitable pressure for absorption by the solid absorbing matrix.

When heating the solid absorbing matrix to release ammonia, it can be that too high ammonia pressure build up occurs inside the system, due to thermal inertia or to a potential failure of the heating power regulation. In order to release the pressure above a given set-point, the excess of gaseous ammonia is preferably released by a safety valve and either directly returned to the ammonia precursor tank (preferred embodiment in the case of a solid ammonia precursor), or first dissolved in an adequate amount of water, for instance coming from the evaporation of the precursor solution the case being, and stored on purpose, and at a composition involving an amount of available ammonia identical to the one of the precursor solution (preferred embodiment in the case of urea precursor solutions). In another preferred embodiment, the excess of ammonia released can merely be dissolved in water and the ammonia solution so obtained can be used later on for thermal ammonia generation and storage on the solid absorbing matrix. This also applies to the case where the solid absorbing matrix releases hydrogen.

The present invention also concerns a system for applying the method as described above, said system comprising:
- a biochemical decomposition unit mounted on board the vehicle and storing at least one protein component adapted to chemically decompose a ammonia precursor for generating a gas;
- at least one power generator mounted on board the vehicle and being configured to convert said gas into power.

In a particular embodiment of the invention, the system comprises:
- a tank mounted on board the vehicle and storing at least one solid absorbing matrix where the gas generated in the biochemical decomposition unit is stored by sorption,
- a tank mounted on board the vehicle and storing ammonia precursor solution;
- means for directing a stream of ammonia precursor solution to the biochemical decomposition unit;
- means for directing the gas (generated in the biochemical decomposition unit) to the solid absorbing matrix.

Preferably, the storage tank for the solid absorbing matrix comprises or is connected to a pressure release valve as described above.

The means for directing the stream of ammonia precursor solution to the biochemical decomposition unit generally comprise a pipe, a valve and eventually a pump, although if the biochemical decomposition unit is located below the storage tank of the ammonia precursor solution, the stream can merely be generated by gravity.

The means for separating the water from the ammonia may be a condenser and/or a membrane as set forth above.

The means for directing the refilling ammonia gas to the storage tank of the solid storage absorbing matrix may be a simple tube (pipe) and said refilling ammonia gas may be mixed with other gaseous decomposition product(s) like CO₂ for instance.

In one embodiment, the system of the invention also comprises a pressure relief valve enabling to release pressure above a given set point in the storage tank of the solid absorbing matrix. Preferably, it also comprises means for dissolving the gases so released into a given amount of water and means for returning the so obtained solution to the storage tank of an ammonia precursor solution.

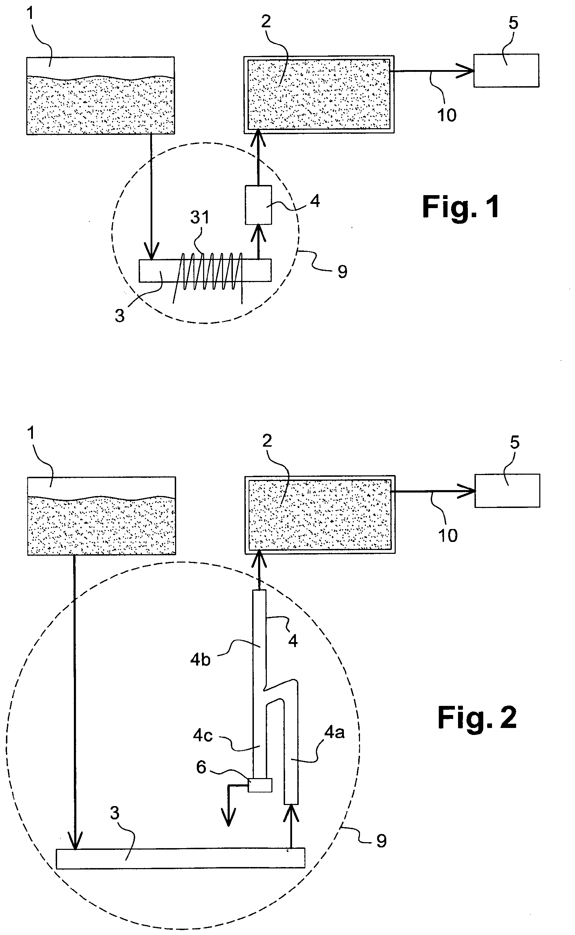

The present invention is illustrated in a non limitative way by the examples below relying on figures 1 to 8 attached. In these figures, identical or similar devices bear identical reference numbers.

### Example 1

Figure 1 is a schematic view of an on board power generation system according to a particular embodiment of the present invention.

As illustrated in Figure 1, a liquid solution of an ammonia precursor is stored in a tank [1] and a solid absorbing matrix is stored in a tank [2]. The tank [1] and the tank [2] are connected together in a communicating manner via a communication line [9]. The communication line [9] comprises a biochemical decomposition unit [3] and a condenser [4].

According to one aspect of the invention, the biochemical decomposition unit [3] receives a stream (i.e. part) of the ammonia precursor. The biochemical decomposition unit [3] contains an enzyme (for example, urease) that catalyzes the hydrolysis (i.e. decomposition) of the ammonia precursor to ammonia. Thus, the biochemical decomposition unit [3] generates the refilling ammonia gas for the regeneration of the solid absorbing matrix.

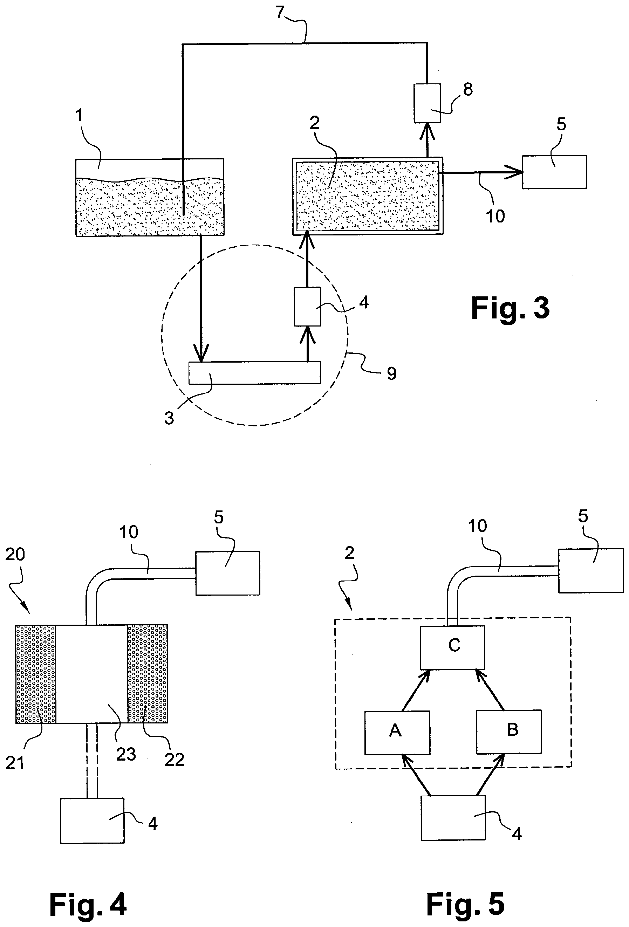

Figure 4 is a schematic view of the tank [2] according to a particular embodiment of the present invention. As illustrated in this example, the tank [2] comprises one cell [20]. The cell [20] comprises two chambers [21] and [22], each containing a solid absorbing matrix. The chambers can contain similar or distinct type of solid absorbing matrix. The chambers [21] and [22] are separated by a gas flow channel [23]. The ammonia gas released from the solid absorbing matrices (contained in the chambers [21] and [22]) and (eventually) the refilling ammonia gas (generated by the biochemical decomposition unit) can flow through the channel [23] towards the condenser [4]. Of course, in another embodiment the cell [20] can comprise one or more than two chamber(s).

According to another particular embodiment of the present invention, the tank [2] can comprise a plurality of cells connected in series and/or in parallel.

Figure 5 is a schematic view of the tank [2] according to another particular embodiment of the present invention. As illustrated in this example, the tank [2] comprises three cells (A, B, C) containing, for example, solid materials showing different ammonia sorption properties. The tank [2] is based on a two-stage unit. The first stage comprises the cells A and B, and the second stage comprises the cell C. For example, the cells A and B are filled with magnesium chloride and the cell C is filled with calcium chloride or barium chloride. One magnesium chloride-filled cell (for example, cell A) is used for the absorption of ammonia generated by the biochemical decomposition unit [3] while the second one (for example, cell B), previously saturated with ammonia (coming from the biochemical decomposition unit [3]), is used to provide ammonia gas which is further absorbed in the cell C. When the cell A is ammonia saturated and the cell B is empty, the roles of cells A and B are reversed. This two-stage unit combines the enhanced absorption properties of one matrix material (for example, magnesium chloride) for ammonia capture, and the advantage of the desorption properties of a second matrix material (for example, calcium chloride or barium chloride) to make ammonia readily available for a power generator [5] or to a chemical converter.

As illustrated in figure 1, the tank [2] is connected to the power generator [5] via a line [10] configured to transport ammonia gas. According to one aspect of the invention, ammonia gas (NH₃) released from the solid absorbing matrix flows through the line [10] and is transferred to the power generator [5] (or a chemical converter).

The liquid solution stored in tank [1] is preferably a 32.5% urea solution commercially available under the brand name Adblue® or a 40% urea solution, but other soluble ammonia compounds (like ammonium carbamate or guanidinium formate) are also suitable. A stream (i.e. part) of the solution enters inside the biochemical decomposition unit [3], where water evaporation and urea decomposition occur. The water is further separated in the condenser [4], and the remaining gaseous flow (i.e. the refilling ammonia gas) goes through the tank [2] where ammonia is trapped on the solid absorbing matrix.

In a particular embodiment, for example when the solid absorbing matrix is saturated with ammonia, a stream (i.e. part) or all of the refilling ammonia gas can flow through the line [10] and be delivered to the power generator [5] (or a chemical converter). In this particular embodiment, the stream or all of the refilling ammonia gas can flow through the solid absorbing matrix, i.e. the stream or all of the refilling ammonia gas is not trapped on the solid absorbing matrix.

As illustrated in figure 1, the biochemical decomposition unit [3] is equipped with a heater [31]. The heater [31] can provide inside the biochemical decomposition unit [3] the optimum temperature for the desired activity of the enzyme. For example, the heat source of the heater [31] can be derived from a hot part of the vehicle, preferably the cooling of the power generator or of electrical batteries. Alternatively, the heater can also be electrical. For example, the temperature range is 30°C - 60°C.

In a particular embodiment, the refilling ammonia gas generated by the biochemical decomposition unit can be compressed to a suitable pressure before absorption by the solid absorbing matrix. To this aim, a gas pressurisation unit (not shown) can be mounted between the biochemical decomposition unit [3] and the condenser [4]. In a particular embodiment, the gas pressurisation unit can comprise a pump. In another embodiment, the gas pressurisation unit can comprise a piston system. In yet another embodiment, the gas pressurisation unit can comprise a controllable valve system.

As regards the content of the tank [2], any material showing ammonia sorption properties is convenient; however, a matrix containing alkaline earth metal chloride is particularly adapted. The excess of carbon dioxide is either trapped in the condensed water or released. Eventually it can be released in the exhaust pipe of the internal combustion engine, if the power generator is an internal combustion engine, when the pressure inside the tank [2] reaches a preset level. In vehicle operation, ammonia is desorbed from the solid which is stored in the tank [2], and carried to the power generator [5] (or a chemical converter). The condensed water can be further vaporized on hot spots of the vehicle such as an exhaust line.

In a particular embodiment, an ammonia adsorption loop can also be used, for example in the form of a convection system with a carrier gas used for ammonia depletion of the biochemical decomposition unit [3], and transfer of the ammonia gas to the solid absorbing matrix. After absorption, the ammonia-free carrier gas is available to be enriched with ammonia by flowing again through the decomposition unit [3].

### Example 2

This example, relying on Figure 2 attached, illustrates the case in which the condenser [4] is made of a shaped tube. The tank [1] is filled with a urea solution. The gas flow resulting from the water evaporation and the urea decomposition goes through the inlet part [4a] of the condenser. Water vapors are condensed in part [4b] of the device, having a temperature lower than part [4a]. Liquid water is further collected in part [4c], and is removed by opening the valve [6]. Ammonia vapor goes to tank [2] through the outlet of the part [4b] of the condenser.

### Example 3

In this example, the condenser of example 1 is removed and the water/gas separation is made effective by using a membrane or a series of membranes at the outlet of the biochemical decomposition unit [3].

### Example 4

In this case, tank [1] is filled with a solid state ammonia precursor, for example: urea or ammonium carbamate, in the form of powder, pellets or flakes. A stream (part) of the solid is drawn to the biochemical decomposition unit [3] where ammonia is generated, and further trapped in a solid material in the tank [2]. No separator device (water condenser, for example) is needed in this example.

### Example 5

In this example, relying on Figure 3, a pressure safety function of tank [2] is described in this example. When pressure build up inside the tank [2] is higher than a set value, the pressure valve [8] is open, and the ammonia gas flows to the tank [1], through the line [7]. Ammonia is further dissolved in the solution which is stored in the tank [1].

In a preferred embodiment, which is not illustrated in Figure 3, line [7] does not return ammonia directly to tank [1] but instead, it conveys it first to a chamber/tank where it is dissolved in an appropriate amount of water so as to reach the right composition (preferably having the same amount of available ammonia as the solution in tank [1] and the urea solution so obtained is then sent to tank [1].

This can be done for instance by storing a given amount of water in the chamber, by deducing the amount of ammonia released from the pressure difference since the beginning of the pressure release and by returning the solution to the tank when the right ammonia concentration is reached.

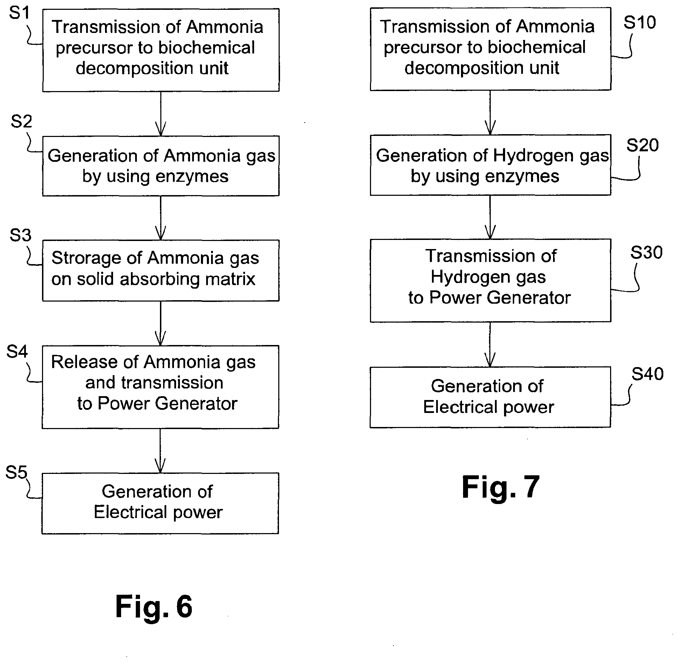

Figure 6 is a schematic view of an on board power generation mechanism, according to a first particular embodiment of the present invention.

At step S1, a stream of ammonia precursor solution is transmitted to a biochemical decomposition unit mounted on board a vehicle.

At step S2, the biochemical decomposition unit generates ammonia gas by chemically decomposing the received ammonia precursor by using enzymes.

At step S3, the ammonia gas is directed to a tank containing a solid absorbing matrix. The ammonia gas is then stored by sorption in the solid absorbing matrix.

At step S4, the solid absorbing matrix is heat-controlled so as to release a desired amount of ammonia gas. The released ammonia gas is then transmitted to a power generator mounted on board the vehicle.

At step S5, the power generator converts the ammonia gas into, for example, an electrical power. The produced electrical power can then be used to recharge batteries mounted on board the vehicle or to power an electrical component or system.

Figure 7 is a schematic view of an on board power generation mechanism, according to a second particular embodiment of the present invention.

At step S10, a stream of ammonia precursor solution is transmitted to a biochemical decomposition unit mounted on board a vehicle.

At step S20, the biochemical decomposition unit generates hydrogen gas by chemically decomposing the received ammonia precursor by using enzymes.

At step S30, the hydrogen gas is transmitted to a power generator mounted on board the vehicle.

At step S40, the power generator converts the hydrogen gas into, for example, an electrical power. The produced electrical power can then be used to recharge batteries mounted on board the vehicle or to power an electrical component or system.

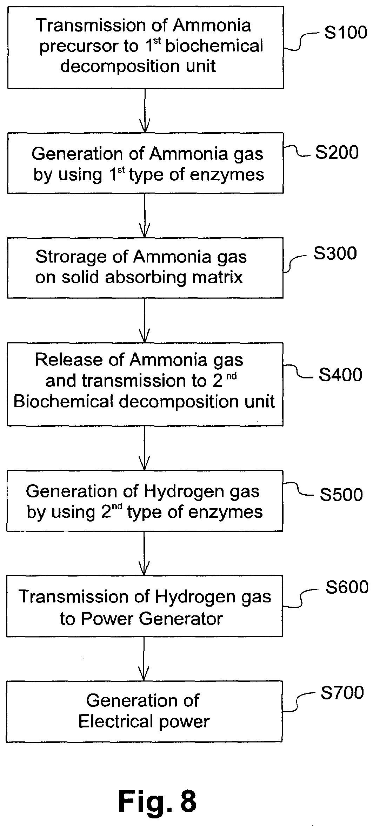

Figure 8 is a schematic view of an on board power generation mechanism, according to a third particular embodiment of the present invention.

At step S100, a stream of ammonia precursor solution is transmitted to a first biochemical decomposition unit mounted on board a vehicle.

At step S200, the first biochemical decomposition unit generates ammonia gas by chemically decomposing the received ammonia precursor by using first type of enzymes.

At step S300, the ammonia gas is directed to a tank containing a solid absorbing matrix. The ammonia gas is then stored by sorption in the solid absorbing matrix.

At step S400, the solid absorbing matrix is heat-controlled so as to release a desired amount of ammonia gas. The released ammonia gas is then transmitted to a second biochemical decomposition unit (or chemical converter) mounted the board a vehicle.

At step S500, the second biochemical decomposition unit generates hydrogen gas by modifying the molecular composition of the received ammonia gas by using second type of enzymes. In other words, step 500 consists in converting NH3 to H2. In another embodiment, at step S500 conventional catalysts such as metal catalyst can also be used for this step instead of biochemical decomposition.

At step S600, the hydrogen gas is transmitted to a power generator mounted on board the vehicle.

At step S700, the power generator converts the hydrogen gas into, for example, an electrical power. The produced electrical power can then be used to recharge batteries mounted on board the vehicle or to power an electrical component or system.

## Claims

1. Method for generating power on board a vehicle, wherein the method comprises:
- generating a gas by chemically decomposing an ammonia precursor in a biochemical decomposition unit (3) mounted on board the vehicle and storing at least one protein component adapted to decompose said ammonia precursor;
- separating and collecting water from the gas generated in the biochemical decomposition unit (3) by means of a liquid-vapour separator unit (4);
- converting said gas into power in at least one power generator (5) mounted on board the vehicle.

2. Method according to claim 1, wherein said gas is an ammonia gas.

3. Method according to claim 1, wherein said gas is an hydrogen gas.

4. Method according to claim 3, wherein the step of generating consists in:
- decomposing the ammonia precursor with a protein component for generating a ammonia gas;
- decomposing the generated ammonia gas for generating the hydrogen gas.

5. Method according to any one of claims 1 to 4, wherein the power generator (5) is a fuel cell.

6. Method according to any one of claims 1 to 4, wherein the power generator (5) is an internal combustion engine.

7. Method according to any one of claims 1 to 6, wherein the gas generated in the biochemical decomposition unit (3) is stored in an intermediate buffer (or tank) (2).

8. Method according to any one of claims 1 to 6, wherein the gas generated in the biochemical decomposition unit (3) is stored by sorption in at least one solid absorbing matrix (2), and wherein the method further comprises a step of releasing the gas from said solid absorbing matrix (2), the released gas being converted into power.

9. Method according to claim 8, wherein the biochemical decomposition unit (3) is equipped with a heater.

10. Method according to any one of claims 1 to 8, wherein said protein component comprises at least one enzyme.

11. Method according to any one of claims 1 to 9, wherein said enzyme is urease.

12. Method according to any one of claims 1 to 11, wherein the ammonia precursor is a solid compound.

13. Method according to any one of claims 1 to 11, wherein the ammonia precursor is an aqueous urea solution.

14. System for generating power on board a vehicle, said system comprising:
- a biochemical decomposition unit (3) mounted on board the vehicle and storing at least one protein component adapted to chemically decompose an ammonia precursor for generating a gas;
- at least one power generator (5) mounted on board the vehicle and being configured to convert said gas into power;
**characterised in that** the system further comprises a liquid-vapour separator unit (4) able to separate and collect water from the gas generated in the biochemical decomposition unit (3).

15. System according to claim 14, wherein it comprises a tank (2) mounted on board the vehicle and storing at least one solid absorbing matrix where the gas generated in the biochemical decomposition unit (3) is stored by sorption.

## Patentansprüche

1. Verfahren zur Energieerzeugung an Bord eines Fahrzeugs, wobei das Verfahren aufweist:
- Erzeugen eines Gases durch chemische Zersetzung einer Ammoniak-Vorstufe in einer biochemischen Zersetzungseinheit (3), welche an Bord des Fahrzeugs angeordnet ist und wenigstens einer Proteinkomponente speichert, welche dazu geeignet ist, die Ammoniak-Vorstufe zu zersetzen;
- Trennen und Sammeln von Wasser von dem in der biochemischen Zersetzungseinheit (3) erzeugten Gas mittels einer Flüssigkeits-Dampf-Trenneinheit (4);
- Umwandeln des Gases in Energie in wenigstens einem Energieerzeuger (5), welcher an Bord des Fahrzeugs angeordnet ist.

2. Verfahren gemäß Anspruch 1, wobei das Gas ein Ammoniakgas ist.

3. Verfahren gemäß Anspruch 1, wobei das Gas ein Wasserstoffgas ist.

4. Verfahren gemäß Anspruch 3, wobei der Schritt des Erzeugens besteht aus:
- Zersetzen der Ammoniak-Vorstufe mit einer Proteinkomponente zur Erzeugung eines Ammoniakgases;
- Zersetzen des erzeugten Ammoniakgases zur Erzeugung des Wasserstoffgases.

5. Verfahren gemäß einem der Ansprüche 1 bis 4, wobei der Energieerzeuger (5) eine Brennstoffzelle ist.

6. Verfahren gemäß einem der Ansprüche 1 bis 4, wobei der Energieerzeuger (5) ein Verbrennungsmotor ist.

7. Verfahren gemäß einem der Ansprüche 1 bis 6, wobei das in der biochemischen Zersetzungseinheit (3) erzeugte Gas in einem Zwischenspeicher (oder Tank) (2) gespeichert wird.

8. Verfahren gemäß einem der Ansprüche 1 bis 6, wobei das in der biochemischen Zersetzungseinheit (3) erzeugte Gas mittels Sorption in wenigstens einer Feststoff-absorbierenden Matrix (2) gespeichert wird, und wobei das Verfahren ferner einen Schritt des Freisetzens des Gases aus der Feststoff-absorbierenden Matrix (2) aufweist, wobei das freigesetzte Gas in Energie umgewandelt wird.

9. Verfahren gemäß Anspruch 8, wobei die biochemische Zersetzungseinheit (3) mit einer Heizung ausgestattet ist.

10. Verfahren gemäß einem der Ansprüche 1 bis 8, wobei die Proteinkomponente wenigstens ein Enzym aufweist.

11. Verfahren gemäß einem der Ansprüche 1 bis 9, wobei das Enzym Urease ist.

12. Verfahren gemäß einem der Ansprüche 1 bis 11, wobei die Ammoniak-Vorstufe eine feste Verbindung ist.

13. Verfahren gemäß einem der Ansprüche 1 bis 11, wobei die Ammoniak-Vorstufe eine wässrige Harnstofflösung ist.

14. System zur Energieerzeugung an Bord eines Fahrzeugs, das System aufweisend:
- eine biochemische Zersetzungseinheit (3), welche an Bord des Fahrzeugs angeordnet ist und wenigstens eine Proteinkomponente speichert, welche dazu geeignet ist, eine Ammoniak-Vorstufe chemisch zu zersetzen, um ein Gas zu erzeugen;
- wenigstens einen Energieerzeuger (5), welcher an Bord des Fahrzeugs angeordnet ist und dazu eingerichtet ist, das Gas in Energie umzuwandeln;
**dadurch gekennzeichnet, dass** das System ferner eine Flüssigkeits-Dampf-Trenneinheit (4) aufweist, welche geeignet ist, Wasser von dem in der biochemischen Zersetzungseinheit (3) erzeugten Gas zu trennen und zu sammeln.

15. System gemäß Anspruch 14, wobei das System einen Tank (2) aufweist, welcher an Bord des Fahrzeugs angeordnet ist und wenigstens eine Feststoffabsorbierende Matrix speichert, in welcher das in der biochemischen Zersetzungseinheit (3) erzeugte Gas mittels Sorption gespeichert wird.

## Revendications

1. Procédé de génération d'énergie à bord d'un véhicule, le procédé comprenant :
- le fait de générer un gaz en décomposant chimiquement un précurseur d'ammoniac dans une unité de décomposition biochimique (3) montée à bord du véhicule et de stocker au moins un composant protéique adapté pour décomposer ledit précurseur d'ammoniac ;
- le fait de séparer l'eau et le gaz générés dans l'unité de décomposition biochimique (3) au moyen d'une unité de séparation liquide-vapeur (4), et de collecter cette eau ;
- le fait de convertir ledit gaz en énergie dans au moins un générateur d'énergie (5) monté à bord du véhicule.

2. Procédé selon la revendication 1, dans lequel ledit gaz est un gaz ammoniac.

3. Procédé selon la revendication 1, dans lequel ledit gaz est un gaz hydrogène.

4. Procédé selon la revendication 3, dans lequel l'étape de génération consiste à:
- décomposer le précurseur d'ammoniac avec un composant protéique pour générer un gaz ammoniac ;
- décomposer le gaz ammoniac généré pour générer le gaz hydrogène.

5. Procédé selon l'une quelconque des revendications 1 à 4, dans lequel le générateur d'énergie (5) est une pile à combustible.

6. Procédé selon l'une quelconque des revendications 1 à 4, dans lequel le générateur d'énergie (5) est un moteur à combustion interne.

7. Procédé selon l'une quelconque des revendications 1 à 6, dans lequel le gaz généré dans l'unité de décomposition biochimique (3) est stocké dans un tampon (ou réservoir) intermédiaire (2).

8. Procédé selon l'une quelconque des revendications 1 à 6, dans lequel le gaz généré dans l'unité de décomposition biochimique (3) est stocké par sorption dans au moins une matrice absorbante solide (2), et dans lequel le procédé comprend en outre une étape consistant à libérer le gaz de ladite matrice absorbante solide (2), le gaz libéré étant converti en énergie.

9. Procédé selon la revendication 8, dans lequel l'unité de décomposition biochimique (3) est équipée d'un dispositif de chauffage.

10. Procédé selon l'une quelconque des revendications 1 à 8, dans lequel ledit composant protéique comprend au moins une enzyme.

11. Procédé selon l'une quelconque des revendications 1 à 9, dans lequel ladite enzyme est une uréase.

12. Procédé selon l'une quelconque des revendications 1 à 11, dans lequel le précurseur d'ammoniac est un composé solide.

13. Procédé selon l'une quelconque des revendications 1 à 11, dans lequel le précurseur d'ammoniac est une solution aqueuse d'urée.

14. Système de génération d'énergie à bord d'un véhicule, ledit système comprenant :
- une unité de décomposition biochimique (3) montée à bord du véhicule et stockant au moins un composant protéique adapté pour décomposer chimiquement un précurseur d'ammoniac pour générer un gaz ;
- au moins un générateur d'énergie (5) monté à bord du véhicule et configuré pour convertir ledit gaz en énergie ;
**caractérisé en ce que** le système comprend en outre une unité de séparation liquide-vapeur (4) apte à séparer l'eau et le gaz générés dans l'unité de décomposition biochimique (3) et à collecter cette eau.

15. Système selon la revendication 14, **caractérisé en ce qu'**il comprend un réservoir (2) monté à bord du véhicule et stockant au moins une matrice absorbante solide dans laquelle le gaz généré dans l'unité de décomposition biochimique (3) est stocké par sorption.
